# EUROPÄISCHE PATENTANMELDUNG

(11) **EP 3 792 926 A1**
(43) Veröffentlichungstag der Anmeldung: **17.03.2021**
(21) Anmeldenummer: 19196873.4
(22) Anmeldetag: 12.09.2019
(51) Int. Cl.: G16H 15/00, G16H 50/30, H04W 4/00

(54) **VERFAHREN UND ANORDNUNG ZUM AUSWERTEN VON DATEN EINES ODER MEHRERER IOT GERÄTE EINES NUTZERS**

(71) Anmelder: Deutsche Telekom AG, 53113 Bonn (DE)
(72) Erfinder: EFFELSBERG, Volker, 53639 Königswinter (DE); HENZE, Thomas, 53173 Bonn (DE)
(74) Vertreter: Raible Deissler Lehmann Patentanwälte PartG mbB

(57) **Zusammenfassung**

Die Erfindung betrifft ein Verfahren zum Auswerten von loT Gerätedaten eines Nutzers, bei dem die loT Gerätedaten von mindestens einem loT Gerät (141, 142, 151, 152, 153) mindestens eines ersten Anbieters erfasst und in einem von einem als Übertragungsnetz implementierten Telekommunikationsnetz bereitgestellten zentralen Datenspeicher (100) gespeichert werden, wobei ein digitaler Dienst (189, 190) mindestens eines zweiten Anbieters nach Autorisierung einen Zugang zu mindestens einer Auswahl von den auf dem zentralen Datenspeicher (100) gespeicherten Daten des mindestens einen loT Geräts (141, 142, 151, 152, 153) erhält und diese abruft und die abgerufenen Daten geräteübergreifend analysiert, auswertet und dem Nutzer (120) bereitstellt. Ferner betrifft die Erfindung eine Anordnung zum Auswerten von loT Gerätedaten eines Nutzers.

## Beschreibung

Die vorliegende Erfindung betrifft ein Verfahren und eine Anordnung zum Auswerten von Daten eines oder mehrerer IoT Geräte eines Nutzers, die bei einer Nutzung der jeweiligen IoT Geräte anfallen und von den jeweiligen IoT Geräten jeweils bereitgestellt bzw. erfasst werden.

IoT (Internet-of-Things) Geräte, im Folgenden auch IoT Devices genannt, finden sich zunehmend im Alltag. Viele davon, wie zum Beispiel "Wearables" (Uhren, Kleidungssensoren, Fitnesstracker, Blutzuckermessgeräte, Herzschrittmacher etc.) sammeln bei ihrer jeweiligen Nutzung eine Reihe von Daten, darunter auch höchst persönliche Daten der Nutzer, im Folgenden auch personenbezogene oder personenbeziehbare Daten genannt. Diese Daten werden in der Regel direkt nach ihrer jeweiligen Erfassung zu einer jeweiligen Cloud-Applikation, in der Regel eines Herstellers des jeweiligen IoT Devices, geschickt, die die Daten aufbereitet und dem Kunden bzw. Nutzer, in der Regel über eine App (kurz für Applikation) oder ein Web-Portal bspw. mittels eines Nutzerendgerätes zur Verfügung stellt. Eine Auswertung und Verarbeitung der Daten, auch der personenbezogenen Daten bleibt demnach in der Regel dem Applikationsanbieter/Hersteller selbst überlassen. So bilden sich sogenannte Insellösungen heraus. Ausnahmen hiervon sind sogenannte "ecosystem-weite" oder interoperable OTT (Over-the-top content) Applikationen, wie bspw. "Apple-Health" (https://www.apple.com/de/ios/health/) oder "Strava" (https://www.strava.com/?hl=de), die in einem fest vorgegebenen Format, Daten von verschiedenen IoT Devices aggregieren können.

Derzeit gibt es für den Kunden bzw. Nutzer keine Möglichkeit, IoT Device Daten über mehrere IoT Geräte beliebig geräteübergreifend miteinander zu verknüpfen, um z.B. Mehrwertdienste freier Drittanbieter zu nutzen. Stattdessen bleiben die Auswertungen der IoT Daten auf das einzelne IoT Gerät des jeweiligen Herstellers beschränkt, es sei denn, es gibt seitens des jeweiligen Herstellers vorgesehene vordefinierte Schnittstellen zu voranstehend genannten Aggregationsanwendungen wie "Apple Health" oder "Strava".

Eine Kombination aus Pulsmessungsdaten eines Nutzers und Fahrzeugbeschleunigungsdaten eines von dem Nutzer gefahrenen Fahrzeugs könnte bspw. Hinweise auf einen emotionalen Zustand eines Autofahrers als Nutzer geben, die dann dem Autofahrer rückgemeldet werden könnten, damit dieser seinen Fahrstil wieder normalisiert. Dies ist heute nicht möglich.

Daten, die durch eine Nutzung von IoT Geräten entstehen und von diesen erfasst werden, sind für einen jeweiligen Nutzer bislang keinen anderen als den von dem Hersteller eines jeweiligen IoT Geräts vorgesehenen Auswertemöglichkeiten zugänglich.

Allerdings ist ein für einen jeweiligen Nutzer nur bedingt kontrollierbarer und nicht selbst steuerbarer Umgang insbesondere mit persönlichen bzw. personenbezogenen Daten für den jeweiligen Nutzer nur wenig komfortabel. Ferner besteht die Gefahr, dass, wenn er bei Nutzung eines jeweiligen IoT Geräts die von dem jeweiligen IoT Gerät erfassten bzw. bereitgestellten IoT Daten von einer Applikation des Herstellers des jeweiligen IoT Gerätes oder von einer der voranstehend genannten bekannten Aggregationsanwendungen auswerten lässt, es dem Nutzer in der Regel nicht klar ist, welche der IoT Daten, insbesondere welche davon ggf. umfassten personenbezogenen Daten von ihm von wem und wo gespeichert werden. So können Profile über den jeweiligen Nutzer immer detaillierter erstellt werden, ohne dass der jeweilige Nutzer eine Kontrolle über seine personenbezogenen Daten hat. Zwar gibt es ein gesetzlich vorgeschriebenes Zweckentfremdungsverbot von personenbezogenen Daten und der Nutzer hat unter bestimmten Voraussetzungen auch das Recht auf Einsicht und Sperrung von personenbezogenen Daten, aber ohne Kenntnis, wo welche Daten hinterlegt sind, hilft dies dem jeweiligen Nutzer nur bedingt.

Mit der bereits existierenden europäischen Datenschutzgrundverordnung werden Regelungen geschaffen, die beschreiben, welche Daten wofür gespeichert werden dürfen. Allerdings wird auch hier nicht das Problem gelöst, dass ein jeweiliger Nutzer selbst eine Übersicht und Kontrolle über seine personenbezogenen Daten verliert, da diese in der Regel über diverse Internet Services verteilt und für den jeweiligen Nutzer schwer einsehbar sind.

Es war somit eine Aufgabe der vorliegenden Erfindung, eine Möglichkeit vorzusehen, einem Nutzer bei Nutzung von IoT Geräten eine Möglichkeit bereitzustellen, die dabei bereitgestellten bzw. erfassten IoT Daten kontrolliert einer von dem Nutzer selbst wählbaren Auswertung bereitzustellen, sei es einer von dem Hersteller eines jeweiligen IoT Geräts vorgesehenen Auswertung, einer voranstehend genannten Aggregationsanwendung oder einer Auswertung eines Drittanbieters.

Zur Lösung des voranstehend genannten Problems stellt die vorliegende Erfindung ein Verfahren und eine Anordnung zum Auswerten von IoT Daten eines Nutzers bei einer Nutzung von IoT Geräten mit den Merkmalen der jeweils unabhängigen Patentansprüche bereit. Weitere Ausgestaltungen sind den jeweiligen abhängigen Ansprüchen und der Beschreibung sowie den Figuren zu entnehmen.

Die vorliegende Erfindung stellt ein Verfahren zum Auswerten von IoT Daten eines Nutzers bereit, bei dem die IoT Daten des Nutzers, die in der Regel auch personenbezogene bzw. personenbeziehbare Daten des Nutzers umfassen, von mindestens einem IoT Gerät mindestens eines ersten Anbieters, bei dessen Nutzung durch den Nutzer, bereitgestellt bzw. erfasst und in einem von einem als Übertragungsnetz implementierten Telekommunikationsnetz bzw. über ein als Übertragungsnetz implementiertes Telekommunikationsnetz bereitgestellten zentralen Datenspeicher gespeichert werden, wobei mindestens ein digitaler Dienst mindestens eines zweiten Anbieters nach Autorisierung einen Zugang zu mindestens einer Auswahl von den auf dem zentralen Datenspeicher gespeicherten IoT Daten des mindestens einen IoT Geräts erhält und diese IoT Daten über eine kommunikative Verbindung abruft und die abgerufenen IoT Daten, insbesondere geräteübergreifend, analysiert, auswertet und dem Nutzer, insbesondere mittels mindestens eines Nutzerendgerätes, bereitstellt.

Die oben genannten IoT Daten eines Nutzers sind Daten, die bei einer durch den Nutzer vorgenommenen Nutzung eines jeweiligen IoT Geräts entstehen bzw. anfallen und von dem jeweiligen IoT Gerät gesammelt, bereitgestellt bzw. erfasst werden. Diese IoT Daten des Nutzers werden im Rahmen der vorliegenden Offenbarung allgemein IoT Daten, IoT Device Daten oder einfach nur IoT Gerätedaten des Nutzers (nämlich des Nutzers, der das jeweilige IoT Gerät benutzt, wenn die Daten anfallen) bezeichnet. Darunter fallen auch bei Nutzung des jeweiligen IoT Geräts vorliegende gerätespezifische Daten, wie bspw. Daten zum Batterie- bzw. Akkustatus. Eine durch den Nutzer vorgenommene Nutzung eines jeweiligen IoT Geräts umfasst dabei auch einen, ggf. längerfristigen oder dauerhaften, Betrieb des IoT Geräts zum Sammeln von IoT Daten für den Nutzer, wie bspw. der Betrieb einer Überwachungskamera, die von dem Nutzer definierte Bereiche überwacht. Demnach können die IoT Daten eines bzw. des Nutzers bzw. die IoT Gerätedaten eines bzw. des Nutzers auch als IoT Daten für einen bzw. den Nutzer bzw. als IoT Gerätedaten für einen bzw. den Nutzer bezeichnet werden.

Bei dem Telekommunikationsnetz kann es sich um ein Mobilfunknetz von einem Mobilfunkbetreiber oder um jede andere Art von Netz handeln, über welches Signale über Kabel, Funk, optisch oder anderweitig elektromagnetisch übertragen werden können.

Die Formulierung "in einem von einem als Übertragungsnetz implementierten Telekommunikationsnetz bereitgestellten zentralen Datenspeicher" bzw. "in einem über ein als Übertragungsnetz implementiertes Telekommunikationsnetz bereitgestellten zentralen Datenspeicher" umfasst dabei sowohl, dass der zentrale Datenspeicher Teil des Telekommunikationsnetzes ist, als auch, dass der zentrale Datenspeicher über mindestens eine Schnittstelle mit dem Telekommunikationsnetz in kommunikativer Verbindung steht und darüber den zentralen Datenspeicher bereitstellt.

In einer möglichen Ausgestaltung ist dabei der zentrale Datenspeicher ein Bestandteil bzw. eine Komponente des Telekommunikationsnetzes, das bspw. als Mobilfunknetz ausgebildet ist. In dazu alternativer Ausgestaltung betreibt ein weiterer Dienstleister den zentralen Datenspeicher, welcher dann über eine oder mehrere geeignete Schnittstellen mit dem jeweiligen Telekommunikationsnetz, insbesondere mit einem jeweiligen Mobilfunknetz in kommunikativer Verbindung steht und darüber sowohl die von dem mindestens einen IoT Gerät des mindestens einen ersten Anbieters erfassten IoT Daten abgespeichert werden als auch nach Autorisierung der Zugang zu den IoT Daten und ggf. das Auslesen der IoT Daten durch den mindestens einen Dienst des mindestens einen zweiten Anbieters ermöglicht wird. So muss nicht jeder Telekommunikationsnetzbetreiber, im Falle eines Mobilfunknetzes nicht jeder Mobilfunkbetreiber seinen eigenen zentralen Datenspeicher betreiben. Ferner kann auch der Nutzer ggf. einen (mobilfunk)netzübergreifenden Betreiber des zentralen Datenspeichers wählen, um z.B. alle seine IoT Device Daten auch bei unterschiedlichen Telekommunikationsverträgen (Mobilfunkverträgen) am gleichen Ort zu bündeln.

Personenbezogene Daten umfassen Daten, die individuell einem jeweiligen Nutzer zugeordnet bzw. zuzuordnen sind. Personenbeziehbare Daten sind Daten, die einem jeweiligen Nutzer prinzipiell aufgrund der Nutzung des jeweiligen IoT Geräts zuordenbar sind, nicht aber unbedingt spezifisch bzw. individuell für den Nutzer sind. Im Folgenden werden personenbezogene und personenbeziehbare Daten zusammengefasst als personenbezogene Daten bezeichnet.

Eine kommunikative Verbindung beschreibt im Rahmen der vorliegenden Offenbarung eine drahtgebundene (wired), eine drahtlose (wireless) oder eine teils drahtgebundene teils drahtlose Verbindung zwischen mindestens zwei Kommunikationspartnern, über welche die Kommunikationspartner, hier das IoT Gerät, das mindestens eine Nutzerendgerät, der jeweilige digitale Dienst und der zentrale Datenspeicher, Daten seitens eines Kommunikationspartners bei dem jeweils anderen an der jeweiligen kommunikativen Verbindung beteiligten Kommunikationspartner abrufen/einsehen, zumindest in eine Richtung übertragen und/oder wechselseitig austauschen können. Die Kommunikationspartner verfügen dabei über jeweilige geeignete Schnittstellen. Bei Verwendung der drahtlosen Verbindung bzw. der teils drahtlosen Verbindung werden im Bereich der drahtlosen Verbindung bzw. Verbindungsabschnitte Signale bzw. Daten per Funk, d. h. durch Modulation von elektromagnetischen Wellen mit Frequenzen unterhalb von sichtbarem Licht übertragen.

Die an dem erfindungsgemäßen Verfahren beteiligten Komponenten bzw. die von der erfindungsgemäßen Anordnung umfassten bzw. damit in Kommunikation stehenden Komponenten sind über jeweilige kommunikative Verbindungen miteinander verbunden bzw. vernetzt. Die Kommunikationspartner verfügen dabei über jeweilige geeignete Schnittstellen.

Ein Vorteil des erfindungsgemäß vorgesehenen bzw. bereitgestellten zentralen Datenspeichers liegt darin, dass ein Nutzer eine Kontrolle über die erfassten IoT Daten, insbesondere über seine personenbezogenen bzw. persönlichen Daten, die durch eine jeweilige Nutzung eines IoT Geräts automatisch entstehen und bei einer jeweiligen Dienstenutzung benötigt und abgerufen werden, erlangt. Dies wird erfindungsgemäß dadurch erreicht, dass die erfassten IoT Daten, insbesondere die personenbezogenen Daten, von einem IoT Gerät und für einen jeweiligen digitalen Dienst bzw. Internet Service zentral an einem Ort, nämlich in dem zentralen Datenspeicher für einen jeweiligen Nutzer, gehalten werden, auf den der jeweilige Nutzer selbst zugreifen kann und einem Dienst seiner Wahl zur Auswertung durch eine geeignet vorgesehene Autorisierung Zugang zu zumindest einer Auswahl von Daten, die auf dem zentralen Datenspeicher für ihn gespeichert sind, gewähren kann, ohne dass der Nutzer dabei auf eine von dem jeweiligen Gerätehersteller vorgesehene und vordefinierte Auswertung, umfassend auch eine durch vordefinierte Schnittstellen ermöglichte Auswertung durch bekannte Aggregationsanwendungen, beschränkt wäre.

Der zentrale Datenspeicher kann bspw. in einer Client-Server-Umgebung oder in einer webbasierten Umgebung, wie einer Cloud-Computing Umgebung Iokalisiert bzw. verortet sein bzw. werden. Der zentrale Datenspeicher ist dabei so konfiguriert, dass einem jeweiligen Nutzer (auf Anfrage) ein oder mehrere jeweilige(r) Speicherbereich(e) zugeordnet ist (sind) bzw. wird (werden), in welchem(n) bei Nutzung eines oder mehrerer IoT Geräte anfallende und erfasste Daten, insbesondere personenbezogene Daten des jeweiligen Nutzers abgelegt bzw. gespeichert werden können.

Ein Kernelement des erfindungsgemäßen Verfahrens ist die Verwendung des zentralen Datenspeichers, in dem alle IoT Daten, insbesondere die von einem jeweiligen IoT Gerät erfassten personenbezogenen Daten, eines jeweiligen Nutzers gehalten, d. h. gespeichert, und dort von dem jeweiligen Nutzer verwaltet, d. h. eingesehen und/oder gelöscht und ggf. auch verändert werden können. IoT Geräte und digitale Dienste, die den erfindungsgemäß verwendeten zentralen Datenspeicher unterstützen, legen dort alle IoT Daten, die sie jeweils für einen jeweiligen Nutzer erfassen, insbesondere personenbezogenen Daten des Nutzers, ab bzw. rufen von dort alle IoT Daten, insbesondere personenbezogene Daten, die sie zur Erbringung des jeweiligen digitalen Dienstes, d. h. einer von dem jeweiligen Nutzer gewünschten Auswertung benötigen, ab. Der jeweilige Dienst kann ggf. auch die bei Durchführung des jeweiligen digitalen Dienstes entstehenden Daten in dem zentralen Datenspeicher abspeichern. Zugleich ist es jedoch erforderlich, dass die digitalen Dienste bzw. die digitalen Dienstleister der jeweiligen digitalen Dienste bzw. die jeweiligen IoT Geräte bzw. deren Hersteller sich dazu verpflichten, IoT Daten, insbesondere personenbezogene Daten, eines jeweiligen Nutzers nicht lokal zu speichern, zumindest nicht dauerhaft, sondern nur nach wiederum erfolgter Freigabe durch den jeweiligen Nutzer von dem zentralen Datenspeicher auslesen.

Gemäß einer Ausführungsform des erfindungsgemäßen Verfahrens ist vorgesehen, dass der Nutzer auf die für ihn gespeicherten IoT Daten eines jeweiligen IoT Gerätes, insbesondere auf die personenbezogenen Daten des Nutzers, zumindest lesend und löschend zugreifen kann.

In weiterer Ausgestaltung des erfindungsgemäßen Verfahrens wird einem jeweiligen IoT Gerät mindestens eine gerätespezifische Sektion bzw. ein gerätespezifischer Sektor in dem zentralen Datenspeicher zur Speicherung der von dem jeweiligen IoT Gerät bereitgestellten bzw. erfassten IoT Daten zugeordnet. Es ist denkbar, dass in einer jeweiligen einem jeweiligen IoT Gerät zugeordneten Sektion, für den jeweiligen das jeweilige IoT Gerät nutzenden Nutzer ein jeweiliger Speicherbereich vorgesehen wird, in dem die von dem jeweiligen IoT Gerät erfassten IoT Daten des Nutzers gespeichert werden, oder dass in einem dem jeweiligen Nutzer in dem Datenspeicher zugeordneten Speicherbereich für ein jeweiliges von dem jeweiligen Nutzer genutztes IoT Gerät eine gerätespezifische Sektion vorgesehen wird.

Dabei erfasst ein jeweiliges IoT Gerät einer Mehrzahl von IoT Geräten jeweilige IoT Daten und speichert die erfassten IoT Daten in der jeweiligen dem jeweiligen IoT Gerät zugeordneten Sektion, insbesondere gerätespezifisch, d. h. bspw. in einem von dem IoT Gerät vorgesehenen Format, ab.

In noch weiterer Ausgestaltung des erfindungsgemäßen Verfahrens ist vorgesehen, dass der digitale Dienst des mindestens einen zweiten Anbieters nach Autorisierung einen Zugang zu den auf dem zentralen Datenspeicher für den Nutzer gespeicherten Daten von einer Auswahl aus der Mehrzahl von IoT Geräten, im Folgenden auch einfach nur Auswahl der IoT Geräte genannt, erhält und zumindest einen Teil der Daten der Auswahl von IoT Geräten aggregiert, verknüpft, auswertet und dem Nutzer, bspw. mittels des Nutzerendgerätes, ein Ergebnis der Auswertung der aggregierten und miteinander verknüpften Daten bereitstellt. Das Ergebnis der Auswertung kann dabei mittels einer Ausgabeeinheit visuell, bspw. mittels eines Displays, und/oder akustisch, bspw. mittels eines Lautsprechers, und/oder haptisch bereitgestellt werden. Ferner kann auch das Ergebnis der Auswertung wiederum in einem dem jeweiligen Dienst zugeordneten Sektor des zentralen Datenspeichers gespeichert werden. Es ist denkbar, dass in einer jeweiligen einem jeweiligen Dienst zugeordneten Sektion, für einen jeweiligen Nutzer ein jeweiliger Speicherbereich vorgesehen wird, in dem die für den jeweiligen Nutzer ausgewerteten Daten gespeichert werden, oder dass in einem dem jeweiligen Nutzer in dem Datenspeicher zugeordneten Speicherbereich einem jeweiligen Dienst eine jeweilige Sektion zugeordnet wird.

Falls der Nutzer zur Auswertung seiner von einem IoT Gerät erfassten IoT Daten eine von dem Hersteller des IoT Gerätes zur Verfügung gestellte Auswertung auswählt, so entspricht der erste Anbieter, d. h. der Hersteller des IoT Gerätes, dem zweiten Anbieter, d. h. dem Anbieter des digitalen Dienstes zur Auswertung.

Falls zur Auswertung notwendig, werden die auszuwertenden Daten temporär bei dem digitalen Dienst zwischengespeichert.

In weiterer Ausgestaltung des erfindungsgemäßen Verfahrens wird die Auswahl der IoT Geräte sowie der von diesen erfassten Daten durch den digitalen Dienst des mindestens einen zweiten Anbieters vorgenommen, insbesondere ist die Auswahl der IoT Geräte bzw. die Art der von den jeweiligen IoT Geräten zu erfassenden bzw. erfassten und für die Auswertung durch den digitalen Dienst notwendigen Daten in dem digitalen Dienst des mindestens einen zweiten Anbieters implementiert.

Durch die sichere zentrale Speicherung der Daten seiner IoT Geräte bzw. IoT Devices in dem zentralen Datenspeicher bekommt der Kunde bzw. Nutzer die Kontrolle über seine Daten, d. h. der erfassten IoT Daten zurück. Er weiß, wo die Daten liegen, kann sie dort einsehen und jederzeit löschen. Zudem bekommt er mehr Freiheit über die Nutzung seiner Daten, indem er Drittanbieter-Applikationen Zugriff auf ausgewählte Sektionen des zentralen Datenspeichers bzw. seines Speicherbereichs auf dem zentralen Datenspeicher gewährt. Damit kann er nicht nur eine Anwendung/Applikation des Herstellers für die Auswertung seiner Daten nutzen (Insellösung), sondern auch alternative Anwendungen von Drittanbietern, die die Daten mehrerer IoT Geräte/loT Devices hersteller- bzw. geräteübergreifend heranziehen, um aus einer neuen Verknüpfung unterschiedlicher Datenquellen neue Erkenntnisse zu gewinnen. Der zentrale Datenspeicher bietet die Möglichkeit der freien Entwicklung einer Vielzahl von Mehrwertdiensten, basierend auf den Daten der IoT Devices, ohne auf die Integration einer vorgegebenen, starren Datenstruktur einer proprietären Plattform angewiesen zu sein, wie dies bei der Bereitstellung von fest vorgegebenen Schnittstellen für die eingangs erwähnten Aggregationsanwendungen, wie Apple Health und Strava der Fall ist.

In noch weiterer Ausgestaltung ist vorgesehen, dass der digitale Dienst des mindestens einen zweiten Anbieters die analysierten und ausgewerteten IoT Daten ebenfalls in dem zentralen, insbesondere in einem dem digitalen Dienst zugeordneten Sektor des zentralen Datenspeichers, insbesondere dienstspezifisch ablegt. Im Rahmen der vorliegenden Offenbarung werden die Begriffe "Sektor" und "Sektion" synonym zueinander verwendet.

Dabei definiert der digitale Dienst des mindestens einen zweiten Anbieters, wie die aggregierten und ausgewerteten Daten des Nutzers in dem dem digitalen Dienst zugeordneten Sektor in dem Sektor gespeichert werden.

Es ist weiterhin vorgesehen, dass neben den von dem mindestens einen IoT Gerät erfassten IoT Daten und ggf. den von dem digitalen Dienst des mindestens einen zweiten Anbieters analysierten und ausgewerteten Daten, die für den Nutzer zumindest lesend und löschend zugänglich gespeichert sind, generische Daten des Nutzers in einem Sektor des zentralen Datenspeichers für den Nutzer lesend, schreibend und löschend zugänglich gespeichert werden.

Im Rahmen der vorliegenden Offenbarung werden der oder die mehreren für den Nutzer vorgesehenen Speicherbereiche auf dem zentralen Datenspeicher auch übergreifend einfach als der zentrale Datenspeicher bezeichnet.

Es ist weiterhin in Ausgestaltung vorgesehen, dass der Nutzer, insbesondere durch eine Nutzereingabe in das mindestens eine Nutzerendgerät, vorgibt, welcher mindestens eine Dienst des mindestens einen zweiten Anbieters durch jeweilige Autorisierung Zugang zu mindestens einer jeweiligen Auswahl von den auf dem zentralen Datenspeicher für den Nutzer gespeicherten Daten des mindestens einen IoT Geräts erhält.

Ein jeweiliger Dienst kann nur während einer aktiven Sitzung des Dienstes auf Anfrage und nach einer Freigabe seitens des den jeweiligen Dienst nutzenden Nutzers über mindestens eine definierte Schnittstelle auf die mindestens eine jeweilige Auswahl von den auf dem zentralen Datenspeicher gespeicherten Daten des mindestens einen IoT Geräts lesend zugreifen.

Die Anfrage seitens des jeweiligen Dienstes wird dem Nutzer auf einer Anzeigeeinheit des Nutzerendgerätes, mit dem der Nutzer den jeweiligen Dienst zuvor über das Telekommunikationsnetz aus dem Internet angefordert hat, angezeigt. Der Nutzer übermittelt mittels des Nutzerendgeräts die Freigabe an den zentralen Datenspeicher. Die Anzeige und/oder die Freigabe können dabei je nach Ausgestaltung des Endgeräts optisch und/oder akustisch erfolgen. Als Endgerät bzw. als Nutzerendgerät kann ein mobiles Endgerät, wie bspw. ein Smartphone, ein Tablet, ein Laptop oder ein PDA, verwendet werden. Aber auch jedes andere geeignete Endgerät kann eingesetzt werden.

In noch einer anderen Ausgestaltung des erfindungsgemäßen Verfahrens generiert der zentrale Datenspeicher nach Eingang der Freigabe für den jeweiligen Dienst einen temporären, d. h. für die Dauer einer jeweilig aktuellen Sitzung ("Session") des jeweiligen Dienstes, Zugangsschlüssel für die mindestens eine jeweilige Auswahl von den auf dem zentralen Datenspeicher gespeicherten Daten des mindestens einen IoT Geräts und übermittelt diesen an das Nutzerendgerät, das den temporären Zugangsschlüssel an den jeweiligen Dienst weiterleitet, wobei der temporäre Zugangsschlüssel nach Beendigung der aktiven Sitzung des jeweiligen Dienstes von dem zentralen Datenspeicher ungültig gemacht wird.

In weiterer Ausgestaltung speichert der jeweilige Dienst zum Abschluss der jeweilig aktuellen Sitzung für eine nachfolgende Sitzung benötigte Daten in dem zentralen Datenspeicher, insbesondere in einem dem jeweiligen Dienst zugeordneten Sektor und löscht alle lokal bei dem jeweiligen Dienst zwischengespeicherten, insbesondere personenbezogenen Daten des Nutzers.

Das bedeutet, dass der zentrale Datenspeicher sowohl für jeweilige IoT Geräte als auch für jeweilige digitale Dienste Schnittstellen anbietet, über die ein jeweiliges IoT Gerät erfasste Daten in dem zentralen Datenspeicher lesen und abspeichern kann und der jeweilige Dienst auf zumindest eine Auswahl der erfassten Daten nach vorangegangener Freigabe durch den jeweiligen Nutzer zur Auswertung der erfassten Daten zugreifen kann. Ein jeweiliger digitaler Dienst darf während der aktiven Nutzungsphase auf durch den Nutzer freigegebene Datenbereiche, d. h. sowohl auf generische Daten des Nutzers, soweit diese ggf. zur Auswertung der auszuwertenden und von dem mindestens einen IoT Gerät erfassten Daten nötig sind, wie auch auf die auszuwertenden und von dem mindestens einen IoT Gerät erfassten Daten zugreifen und nach Abschluss der Nutzung Daten für die nächste Nutzung dort ablegen, insbesondere in einem dem jeweiligen Dienst zugeordneten Sektor.

Ferner betrifft die vorliegende Erfindung eine Anordnung zum Auswerten von bei durch einen Nutzer vorgenommenen Nutzung mindestens eines IoT Geräts entstandenen IoT Daten, mit mindestens einem IoT Gerät eines ersten Anbieters, das dazu konfiguriert ist, die IoT Daten, insbesondere personenbezogene Daten des Nutzers zu erfassen und in einem von einem als Übertragungsnetz implementierten Telekommunikationsnetz bereitgestellten zentralen Datenspeicher zu speichern, mit dem zentralen Datenspeicher, der dazu ausgelegt ist, die von dem mindestens einen IoT Gerät erfassten Daten zu speichern und mindestens eine Auswahl der gespeicherten Daten für mindestens einen digitalen Dienst von mindestens einem zweiten Anbieter nach Autorisierung abrufbar zugänglich zu machen.

In möglicher Ausgestaltung umfasst die erfindungsgemäße Anordnung den mindestens einen digitalen Dienst des mindestens einen zweiten Anbieters, wobei der digitale Dienst dazu konfiguriert ist, nach Autorisierung einen Zugang zu der mindestens einen Auswahl von den auf dem zentralen Datenspeicher gespeicherten Daten des mindestens einen IoT Geräts zu erhalten und die Auswahl von Daten abzurufen und die abgerufenen Daten, insbesondere geräteübergreifend, zu analysieren, auszuwerten und dem Nutzer, bspw. mittels eines Nutzerendgerätes, bereitzustellen.

Gemäß einer weiteren Ausgestaltung der erfindungsgemäßen Anordnung ist der zentrale Datenspeicher dazu ausgelegt, für einen jeweiligen Nutzer eine Mehrzahl von Sektoren vorzusehen, wobei jeder Sektor einem jeweiligen IoT Gerät des mindestens einen loT Geräts, das von dem jeweiligen Nutzer genutzt wird, zuordenbar ist, und von einem jeweiligen IoT Gerät, bei seiner Nutzung durch den jeweiligen Nutzer, erfasste IoT Daten in dem jeweiligen dem jeweiligen IoT Gerät zugeordneten Sektor abzuspeichern.

Ferner ist gemäß noch weiterer Ausgestaltung der erfindungsgemäßen Anordnung der zentrale Datenspeicher dazu konfiguriert, nach Eingang einer Freigabe für einen jeweiligen Dienst einen temporären, d. h. für die Dauer einer jeweilig aktuellen Sitzung des jeweiligen Dienstes, Zugangsschlüssel für von dem jeweiligen Dienst angefragte IoT Daten des Nutzers, insbesondere personenbezogene Daten zu generieren und an das Nutzerendgerät des Nutzers zu übermitteln, das den temporären Zugangsschlüssel an den jeweiligen Dienst weiterleitet, und den temporären Zugangsschlüssel nach Beendigung der Sitzung für einen jeweiligen Dienst ungültig zu machen.

In noch fortgeführter Ausgestaltung der erfindungsgemäßen Anordnung sind generische Daten des Nutzers in einem weiteren Sektor des zentralen Datenspeichers für den Nutzer lesend, schreibend und löschend zugänglich gespeichert. Das bedeutet, dass der Nutzer die Möglichkeit hat, auf die seine Person betreffenden allgemein gültigen Daten sowohl lesend als auch schreibend und löschend zuzugreifen. Er kann somit sein digitales Erscheinungsbild, was durch die generischen Daten zum Ausdruck kommen soll, durch einen schreibenden Zugriff verändern bzw. aktiv beeinflussen. Somit kann er aktiv sein Recht auf informationelle Selbstbestimmung ausüben.

In dem jeweiligen einem jeweiligen IoT Gerät zugeordneten Sektor des zentralen Datenspeichers für den jeweiligen Nutzer darf ein jeweiliger Nutzer auf seine personenbezogenen Daten, zumindest lesend und löschend zugreifen.

Die generischen Daten, die in dem weiteren Sektor für den jeweiligen Nutzer in dem zentralen Datenspeicher gespeichert sind bzw. werden, enthalten allgemein persönliche Daten des jeweiligen Nutzers, wie bspw.:
- 1.: Name
- 2.: Adresse
- 3.: Alter
- 4.: Geschlecht
- 5.: Materialstatus
- 6.: Finanzstatus
- 7.: Beschäftigungsstatus
- 8.: Arbeitgeber
- 9.: Portraitbild
- 10.: Freunde
- 11.: Beziehungen
- 12.: Aktivitäten
- 13.: Interessen

Diese generischen Daten können auf Seiten des Nutzers selbst geschrieben, gelesen und aus dem dem Nutzer zugeordneten weiteren Sektor des zentralen Datenspeichers gelöscht werden.

In noch fortgesetzt weiterer Ausgestaltung der erfindungsgemäßen Anordnung ist für einen jeweiligen Nutzer für eine Anzahl von von dem Nutzer genutzten IoT Geräten eine gleiche Anzahl von Sektoren bereitgestellt und jeweils ein Sektor jeweils einem jeweiligen dieser IoT Geräte der Anzahl von IoT Geräten zugeordnet, wobei der dem jeweiligen IoT Gerät zugeordnete Sektor ausgebildet ist, die von dem jeweiligen IoT Gerät erfassten Daten zu speichern, wobei ein jeweiliges IoT Gerät definiert, welche Daten des Nutzers und wie die erfassten Daten des Nutzers in dem jeweiligen dem jeweiligen IoT Gerät zugeordneten Sektor in dem mindestens einen Sektor zu speichern sind. Eine Zuordnung eines jeweiligen Sektors zu einem jeweiligen IoT Gerät erfolgt dynamisch, d. h. immer dann, wenn der Nutzer das jeweilige IoT Gerät nutzt und eine Speicherung der von dem jeweiligen IoT Gerät für den Nutzer erfassten Daten für den Nutzer in dem zentralen Datenspeicher bei dem zentralen Datenspeicher, bspw. über eine Applikation mittels eines Nutzerendgeräts anfordert.

Der zentrale Datenspeicher weist mindestens eine erste Schnittstelle auf, über welche das mindestens eine IoT Gerät des ersten Anbieters von ihm erfasste Daten an den zentralen Datenspeicher übertragen und dort in einem, insbesondere dem mindestens einen IoT Gerät jeweils zugeordneten Sektor speichern kann. Ferner weist der zentrale Datenspeicher mindestens eine zweite Schnittstelle auf, über welche der mindestens eine digitale Dienst des mindestens einen zweiten Anbieters nach Autorisierung von dem mindestens einen digitalen Dienst auszuwertende und von dem mindestens einen IoT Gerät erfasste Daten abrufen kann.

Darüber hinaus betrifft die vorliegende Erfindung ein Computerprogrammprodukt mit einem computerlesbaren Medium und einem auf dem computerlesbaren Medium gespeicherten Computerprogramm mit Programmcodemitteln, die dazu konfiguriert sind, bei Ablauf des Computerprogramms auf einer Recheneinheit, das voranstehend beschriebene erfindungsgemäße Verfahren, insbesondere unter Verwendung der erfindungsgemäßen Anordnung auszuführen.

Das hier im Rahmen der vorliegenden Offenbarung beschriebene Computerprogramm kann in einem internen Computerspeicher oder in einem nichtflüchtigen computerlesbaren Medium gespeichert bzw. abrufbar hinterlegt sein.

Jedes von dem Nutzer genutzte IoT Gerät bekommt für den Nutzer einen eigenen Daten-Sektor auf dem zentralen Datenspeicher bzw. -server, die es mit IoT Gerätedaten, insbesondere auch mit personenbezogenen Daten des Nutzers beschreiben darf. Der Kunde bzw. Nutzer wählt selbst, durch welche App, d. h. Anwendung bzw. Applikation, seine Daten ausgewertet und zur Anzeige gebracht werden, i.e. durch eine OEM-APP des IoT Geräteherstellers oder wahlweise durch eine alternative Drittanbieter-Applikation.

Dabei wird erfindungsgemäß sichergestellt, dass unter anderem die persönlichen Daten, d. h. die personenbezogenen und personenbeziehbaren Daten des Nutzers, die von dem IoT Gerät bzw. IoT Device, bei Nutzung durch den Nutzer, erfasst werden, unter der Kontrolle des Nutzers bleiben. Der Nutzer entscheidet darüber, wie und in welcher Weise er oder andere auf diese Daten zugreifen können und er ist in der Lage, die Daten jederzeit zu löschen.

Die Integration der IoT Gerätedaten in KI Auswertesysteme (KI = künstliche Intelligenz) obliegt einem jeweiligen Drittanbieter, der eine Auswertung der von dem IoT Gerät erfassten Daten anbietet, und ist nicht mehr Aufgabe des IoT Geräte-Herstellers, wie es derzeit bei Integration bspw. in "Apple Health" oder "Strava" notwendig ist. So kann ein freier Markt für KI basierte Mehrwertdienste auf Basis von IoT Gerätedaten entstehen.

Weitere Vorteile und Ausgestaltungen der Erfindung ergeben sich aus der Beschreibung und der beiliegenden Zeichnung.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird anhand eines Ausführungsbeispiels in der Zeichnung schematisch dargestellt und wird im Folgenden unter Bezugnahme auf die Zeichnung ausführlich beschrieben.
Figur 1 zeigt in schematischer Darstellung eine Ausführungsform der erfindungsgemäß vorgesehenen bzw. bereitgestellten Anordnung.
Figur 2 zeigt ein Flussdiagramm für eine mögliche Nutzung einer Ausführungsform der erfindungsgemäßen Anordnung.

Die Figuren werden figurenübergreifend beschrieben, gleichen Komponenten bzw. Verfahrensschritten sind gleiche Bezugszeichen zugeordnet.

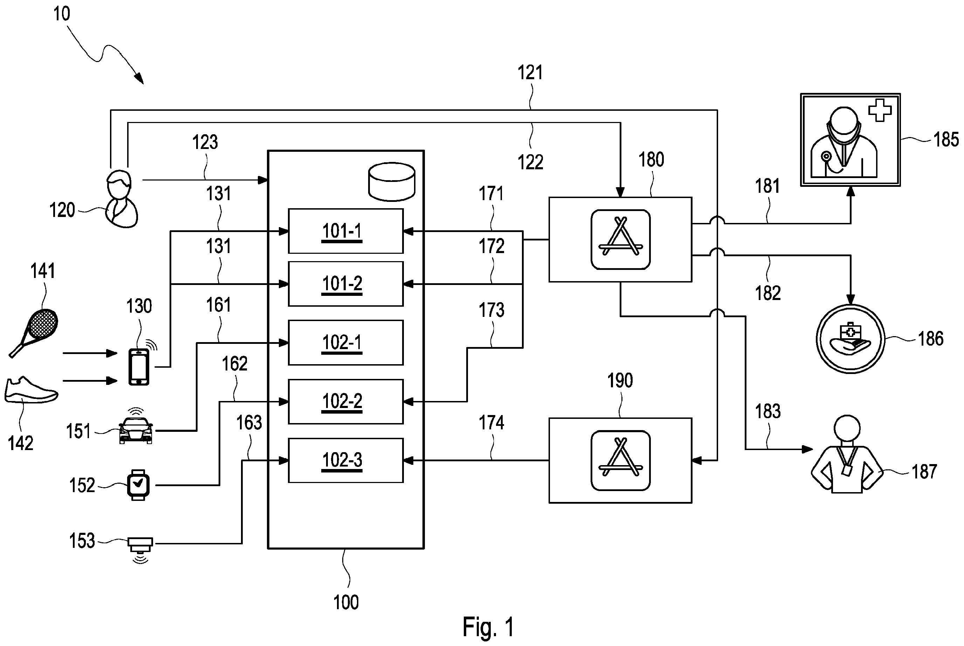

In Figur 1 ist eine Ausführungsform einer erfindungsgemäßen Anordnung 10 gezeigt. Die Anordnung 10 umfasst einen zentralen Datenspeicher 100 und eine Mehrzahl von IoT Geräten 141, 142, 151, 152, 153, die jeweilig von einem jeweiligen ersten Anbieter bzw. Hersteller bereitgestellt werden. Ferner gezeigt ist ein Nutzer 120 und ein Nutzerendgerät 130. Darüber hinaus ist ein digitaler Dienst 180 eines zweiten Anbieters bzw. eines Drittanbieters gezeigt. Für das IoT Gerät 153 ist auch ein herstellerspezifischer digitaler Dienst 190 zur Auswertung der von dem IoT Gerät 153 erfassten Daten gezeigt. Als externe Personen bzw. Institutionen sind ein Arzt 185, eine Krankenversicherung 186 und ein Fitnesscoach bzw. -trainer 187 dargestellt. All diese Komponenten bzw. Personen und/oder Institutionen sind mittelbar über den zentralen Datenspeicher 100 oder unmittelbar miteinander über jeweilige kommunikative Verbindungen miteinander vernetzt.

Der Tennisschläger 141 ist über eine geeignete Funktechnologie, bspw. über Bluetooth mit dem Nutzerendgerät 130 gekoppelt. Mittels einer auf dem Nutzerendgerät 130 implementierten Applikation können IoT Daten von dem Tennisschläger 141 erfasst und über das Nutzerendgerät 130 und dessen kommunikative Verbindung 131 zu dem zentralen Datenspeicher 100 übermittelt und dort für den Nutzer 120 in einem dem Tennisschläger 141 zugeordneten Sektor 101-1 gespeichert werden. Ebenso können mittels einer weiteren oder der gleichen auf dem Nutzerendgerät 130 implementierten Applikation IoT Daten von einem Turnschuh 142 erfasst und über das Nutzerendgerät 130 und dessen kommunikative Verbindung 131 zu dem zentralen Datenspeicher 100 übermittelt und dort für den Nutzer 120 in einem dem Turnschuh 142 zugeordneten Sektor 101-2 gespeichert werden. Um die jeweils erfassten Daten in dem zentralen Datenspeicher 100 abspeichern zu können, muss sich das Nutzerendgerät 130 über die kommunikative Verbindung 131 bzw. bereits zu deren Aufbau bei dem zentralen Datenspeicher 100 jeweils authentifizieren. Erst dann können die erfassten Daten in die dazu vorgesehenen Sektoren 101-1 bzw. 101-2 geschrieben, d. h. abgespeichert werden.

Ein Auto 151 als weiteres IoT Gerät kann ferner über eine kommunikative Verbindung 161, zu deren Aufbau und/oder Nutzung sich das Auto 151 erst authentifizieren muss, von dem Auto 151 für den Nutzer 120 erfasste Daten, wie Geschwindigkeitsdaten, etc. an den zentralen Datenspeicher 100 übertragen und dort in einem dem Auto 151 zugeordneten Sektor 102-1 für den Nutzer 120 abspeichern.

Ebenso kann eine Uhr des Nutzers 120, bspw. eine Smartwatch/Wearable 152 als weiteres IoT Gerät über eine kommunikative Verbindung 162, zu deren Aufbau und/oder Nutzung sich die Uhr 152 ebenfalls erst authentifizieren muss, von der Uhr 152 für den Nutzer 120 erfasste IoT Daten, wie Puls des Nutzers 120, Schrittzahl des Nutzers 120 etc. an den zentralen Datenspeicher 100 übertragen und dort in einem der Uhr 152 zugeordneten Sektor 102-2 für den Nutzer 120 abspeichern.

Ferner gezeigt ist eine (Überwachungs-)Kamera 153 des Nutzers 120 als weiteres IoT Gerät, das über eine kommunikative Verbindung 163, zu deren Aufbau und/oder Nutzung sich die Kamera 153 ebenfalls erst authentifizieren muss, von der Kamera 153 für den Nutzer 120 erfasste IoT Daten, wie Bewegungsabläufe des Nutzers etc. an den zentralen Datenspeicher 100 übertragen und dort in einem der Kamera 153 zugeordneten Sektor 102-3 für den Nutzer 120 abspeichern.

Der Nutzer 120 hat generell, bspw. mittels seines Nutzerendgeräts 130 über eine kommunikative Verbindung 123 zu dem zentralen Datenspeicher 100 die Möglichkeit seine auf dem zentralen Datenspeicher 100 hinterlegten IoT Daten, die von den einzelnen IoT Geräten erfasst und in den jeweiligen ihnen jeweils zugeordneten Sektoren abgelegt sind, zu lesen und/oder zu löschen.

Ferner kann der Nutzer 120, bspw. ebenfalls über sein Nutzerendgerät 130 auswählen, ob er die IoT Daten, darunter ggf. auch seine personenbezogenen Daten, die von einem jeweiligen von ihm genutzten IoT Gerät erfasst wurden, von einer jeweiligen geräteherstellereigenen Auswerteapplikation und/oder von einem Drittanbieter auswerten lassen möchte. Für die von den IoT Geräten 141, 142 und 151 erfassten Daten, die in den jeweiligen den jeweiligen IoT Geräten zugeordneten Sektoren 101-1, 101-2, 102-1 abgelegt sind, wählt der Nutzer 120 hier bspw. den Drittanbieter 180, der über eine kommunikative Verbindung 171 auf den Sektor 101-1, über eine kommunikative Verbindung 172 auf den Sektor 101-2 und über eine kommunikative Verbindung 173 auf den Sektor 102-1 zugreifen kann. Dafür muss sich der Drittanbieter 180 bzw. der von ihm bereitgestellte digitale Dienst allerdings erst authentifizieren bzw. durch den Nutzer 120 autorisiert werden. Der Drittanbieter 180 kann nun diese Daten bzw. eine Auswahl davon abrufen, aggregieren und gemeinsam auswerten. Ein Ergebnis der Auswertung kann sowohl dem Nutzer 120 als auch, nach Autorisierung durch den Nutzer 120, anderen Institutionen, wie dem Arzt 185, der Krankenversicherung 186 und/oder dem Fitnesstrainer 187 bereitgestellt werden. Diese können das Ergebnis jeweils über eine jeweilige kommunikative Verbindung 181, 182, 183 und eine geeignete Ausgabeeinheit angezeigt bekommen - dabei kann es sich bspw. um eine akustische, optische und/oder eine kombinierte akustisch-optische Ausgabe handeln.

In dem hier gezeigten Beispiel werden die von der Kamera 153 erfassten Daten, die in dem Sektor 102-3 auf dem zentralen Datenspeicher 100 gespeichert sind, von einer geräte- bzw. herstellereigenen Auswerteanwendung 190 über eine kommunikative Verbindung 174 ausgewertet. Im Gegensatz zu dem Drittanbieter 180, der über seine jeweiligen kommunikativen Verbindungen 171, 172, 173 auf die Daten nur lesend zugreifen kann, kann die Auswerteanwendung 190 nach Freigabe durch den Nutzer 120 auf die Daten sowohl lesend, als auch schreibend und sogar löschend zugreifen. Auch die geräte- bzw. herstellereigene Auswerteanwendung 190 stellt ein Ergebnis der Auswertung dem Nutzer 120 bereit, das dieser über eine kommunikative Verbindung 121 und ein geeignetes Ausgabegerät einsehen kann.

Die hier gezeigte Anordnung 10 kann bspw. für eine Gesundheitsüberwachung. auch "health monitoring" genannt, verwendet werden. Die Gesundheitsüberwachung wird dabei bspw. durch Kombination aus Tennisschlägerdaten des Tennisschlägers 141, Bewegungsdaten des Tennisschuhs 142 und Pulsdaten der Pulsuhr 152 verbessert. Es wird deutlich, wie genau sich Bewegung durch Laufen und Tennisschlägen auf den Puls auswirken. Damit lassen sich Rückschlüsse über Fitness und Trainingszustand ziehen und daraus konkrete Anleitungen zur Verbesserung oder Stabilisierung des Fitnesszustandes machen und/oder es lassen sich auch ggf. gesundheitliche Alarme ableiten (z.B. hoher Puls ohne aktuelle Bewegung nach körperlicher Anstrengung -> Kreislaufprobleme). Hierzu hat der Entwickler der Drittanbieter-App 180 vorab die Datenformate der IoT Geräte 141, 142 und 152 für sich analysiert und alle relevanten Datenattribute für seine Auswertung eingebunden (Integration). Dadurch ist er in der Lage, die für seine Auswertung notwendigen Daten der IoT Geräte 141, 142, 152 von dem zentralen Datenspeicher abzurufen und durch geeignete Aggregation möglicherweise Erkenntnisse aus der Kombination der Daten mehrerer IoT Geräte/Devices zu gewinnen und daraus Mehrwertdienste zu generieren. Der jeweilige IoT Geräte-Hersteller muss sich dabei nicht um eine Integration seiner Datenformate in unterschiedliche Plattformen kümmern.

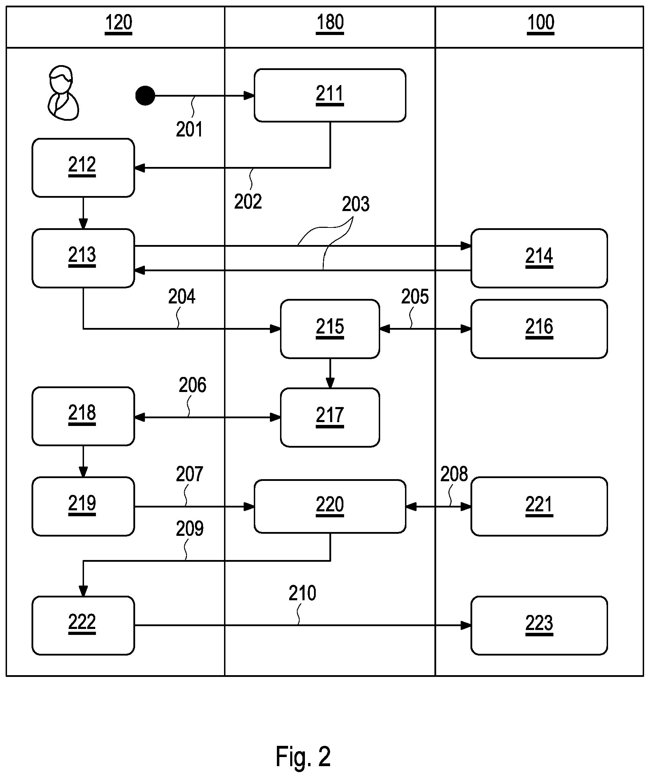

Figur 2 zeigt ein Flussdiagramm für eine beispielhafte Nutzung mehrere IoT Geräte zur Erfassung von IoT Daten eines Nutzers, insbesondere personenbezogenen Daten des Nutzers und eines digitalen Dienstes zur Auswertung der personenbezogenen Daten unter Verwendung einer Ausführungsform der erfindungsgemäßen Anordnung mit dem zentralen Datenspeicher.

Kunde bzw. Nutzer 120 beschließt mit Schritt 201, den digitalen Dienst 180 zur Auswertung der Daten zu nutzen und besucht dazu die dem Dienst 180 zugeordnete Internetseite bzw. Webseite. Der Dienst 180 generiert daraufhin in einem Schritt 211 eine Datenzugriffsanfrage für zur Ausführung des Dienstes benötigte IoT Daten, wie bspw. Zugriff auf die in den den jeweiligen IoT Geräten 141, 142, 152 zugeordneten Sektoren des zentralen Datenspeichers 100 hinterlegten Daten des Nutzers 120 und/oder auf die in einem weiteren Sektor des zentralen Datenspeichers 100 hinterlegten generischen Daten des Nutzers 120, wie Name, Adresse und Alter des Nutzers 120. In einem Schritt 202 übermittelt er diese Anfrage an das Endgerät 130 des Nutzers 120. Das Endgerät 130 zeigt auf einem ihm zugeordneten Display in einem Schritt 212 dem Nutzer 120 die von dem digitalen Dienst 180 gestellte Anfrage an, sodass der Nutzer 120 eine Auswahl zwischen Freigabe und Nichtfreigabe hat. Wählt der Nutzer 120 die Freigabe, so generiert er in Schritt 213 eine Berechtigungsanfrage für einen Datenzugriff auf den zentralen Datenspeicher 100. Diese Berechtigungsanfrage wird von dem Endgerät 130 bzw. dem Nutzer 120 in einem Schritt 203 an den zentralen Datenspeicher 100 geschickt. Der Datenspeicher 100 wertet in Schritt 214 die Nutzeranfrage aus, generiert einen Zugangsschlüssel für den anfragenden Dienst 180 zu seitens des Dienstes angeforderten Daten des Nutzers 120, die entweder im weiteren Sektor oder in dem den IoT Geräten 141, 142, 152 zugeordneten Sektoren des zentralen Datenspeichers 100 hinterlegt sind, und sendet den generierten Zugangsschlüssel wiederum im gleichen Austauschvorgang 203 an das Endgerät 130 des Nutzers 120 zurück. Das Endgerät 130 sendet nunmehr den temporären Zugangsschlüssel in einem Schritt 204 an den Dienst 180, der den Zugangsschlüssel in einem Schritt 215 empfängt und in einem Schritt 205 die von ihm gewünschten und benötigten Daten zur Ausführung des Dienstes bzw. der Auswertung auf dem zentralen Datenspeicher 100 abruft. Dabei ist es denkbar, dass er in einem Schritt 216 generische Daten aus dem weiteren Sektor des zentralen Datenspeichers 100 liest, wie bspw. Name, Adresse und Alter des Nutzers 120, und ferner Daten aus den den IoT Geräten 141, 142, 152 zugeordneten Sektoren liest. Unter Nutzung der Daten ist der Dienst 180 nunmehr in der Lage, in einem Schritt 217 eine auf den Nutzer 120 zugeschnittene Auswertung vorzunehmen und dem Nutzer 120 in einem Schritt 206 bereitzustellen, sodass der Nutzer 120 das Ergebnis in einem Schritt 218 einsehen kann. Wenn der Nutzer 120 in einem Schritt 219 die Sitzung bzw. die Nutzung des Dienstes beenden will, so teilt er dies in einem Schritt 207 dem Dienst 180 mit. Der Dienst 180 entscheidet dabei in einem Schritt 220, welche Daten er für eine zukünftige Sitzung bzw. Nutzung für den Nutzer auf dem zentralen Datenspeicher 100 speichern möchte und löscht alle lokalen, d. h. lokal bei dem Dienst 180 gespeicherten Sitzungsdaten. Die Daten, die er auf dem zentralen Datenspeicher 100 speichern möchte, überträgt er in einem Schritt 208 mittels des Zugangsschlüssels an den zentralen Datenspeicher 100, sodass die relevanten Daten in einem Schritt 221 in einem dem Dienst 180 zugeordneten Sektor des zentralen Datenspeichers 100 abgespeichert werden. Dann bestätigt der Dienst 180 gegenüber dem Endgerät 130 bzw. dem Nutzer 120 in einem Schritt 209, dass die Sitzung beendet wird, sodass der Nutzer 120 in einem Schritt 222 daraufhin beschließt, die Sitzung auch gegenüber dem zentralen Datenspeicher 100 zu beenden, was er dem zentralen Datenspeicher 100 in einem Schritt 210 mitteilt, sodass dieser in einem Schritt 223 den temporären Zugangsschlüssel für den Dienst 180 ungültig macht.

### Bezugszeichenliste

- 10: Anordnung
- 100: zentraler Datenspeicher
- 101-1: Tennisschläger zugeordneter Sektor
- 101-2: Turnschuh zugeordneter Sektor
- 102-1: Auto zugeordneter Sektor
- 102-2: Uhr zugeordneter Sektor
- 102-3: Kamera zugeordneter Sektor
- 120: Nutzer
- 121: kommunikative Verbindung
- 122: kommunikative Verbindung
- 123: kommunikative Verbindung
- 130: Nutzerendgerät
- 131: kommunikative Verbindung
- 141: Tennisschläger als IoT Gerät
- 142: Turnschuh als IoT Gerät
- 151: Auto als IoT Gerät
- 152: Uhr als IoT Gerät
- 153: Kamera als IoT Gerät
- 161: kommunikative Verbindung
- 162: kommunikative Verbindung
- 163: kommunikative Verbindung
- 171: kommunikative Verbindung
- 172: kommunikative Verbindung
- 173: kommunikative Verbindung
- 174: kommunikative Verbindung
- 180: digitaler Dienst
- 181: kommunikative Verbindung
- 182: kommunikative Verbindung
- 183: kommunikative Verbindung
- 185: Arzt
- 186: Krankenkasse
- 187: Fitnesstrainer
- 190: herstellerspezifischer digitaler Dienst
- 201 - 223: Verfahrensschritte

## Patentansprüche

1. Verfahren zum Auswerten von IoT Gerätedaten eines Nutzers, bei dem die IoT Gerätedaten von mindestens einem IoT Gerät (141, 142, 151, 152, 153) mindestens eines ersten Anbieters erfasst und in einem von einem als Übertragungsnetz implementierten Telekommunikationsnetz bereitgestellten zentralen Datenspeicher (100) gespeichert werden, wobei ein digitaler Dienst (189, 190) mindestens eines zweiten Anbieters nach Autorisierung einen Zugang zu mindestens einer Auswahl von den auf dem zentralen Datenspeicher (100) gespeicherten Daten des mindestens einen IoT Geräts (141, 142, 151, 152, 153) erhält und diese abruft und die abgerufenen Daten geräteübergreifend analysiert, auswertet und dem Nutzer (120) bereitstellt.

2. Verfahren nach Anspruch 1, bei dem der Nutzer (120) auf die gespeicherten IoT Gerätedaten zumindest lesend und löschend zugreifen kann.

3. Verfahren nach Anspruch 1 oder 2, bei dem einem jeweiligen IoT Gerät (141, 142, 151, 152, 153) eine gerätespezifische Sektion (101-1, 101-2, 102-1, 102-2, 102-3) in dem zentralen Datenspeicher (100) zur Speicherung der von dem jeweiligen IoT Gerät (141, 142, 151, 152, 153) erfassten IoT Gerätedaten zugeordnet wird.

4. Verfahren nach Anspruch 3, bei dem ein jeweiliges IoT Gerät (141, 142, 151, 152, 153) einer Mehrzahl von IoT Geräten (141, 142, 151, 152, 153) jeweilige IoT Gerätedaten erfasst und in der jeweiligen dem jeweiligen IoT Gerät (141, 142, 151, 152, 153) zugeordneten Sektion (101-1, 101-2, 102-1, 102-2, 102-3), insbesondere gerätespezifisch abspeichert.

5. Verfahren nach Anspruch 4, bei dem der digitale Dienst (189, 190) des mindestens einen zweiten Anbieters nach Autorisierung einen Zugang zu den auf dem zentralen Datenspeicher (100) gespeicherten Daten von einer Auswahl aus der Mehrzahl von IoT Geräten (141, 142, 151, 152, 153) erhält und zumindest ein Teil der Daten der Auswahl von IoT Geräten (141, 142, 151, 152, 153) aggregiert, verknüpft, auswertet und dem Nutzer (120) mittels eines Nutzerendgerätes (130) ein Ergebnis der Auswertung der aggregierten und miteinander verknüpften Daten bereitstellt.

6. Verfahren nach Anspruch 4 oder 5, bei dem die Auswahl der Mehrzahl der IoT Geräte (141, 142, 151, 152, 153) durch den digitalen Dienst (189, 190) des mindestens einen zweiten Anbieters vorgenommen wird, insbesondere in dem digitalen Dienst (189, 190) des mindestens einen zweiten Anbieters implementiert ist.

7. Verfahren nach einem der voranstehenden Ansprüche, bei dem der Nutzer, insbesondere durch eine Nutzereingabe in das mindestens eine Nutzerendgerät, vorgibt, welcher mindestens eine Dienst des mindestens einen Anbieters durch jeweilige Autorisierung Zugang zu mindestens einer jeweiligen Auswahl von den auf dem zentralen Datenspeicher (100) gespeicherten Daten des mindestens einen IoT Geräts erhält.

8. Verfahren nach Anspruch 7, bei dem ein jeweiliger Dienst (180, 190) nur während einer aktiven Sitzung des Dienstes (180, 190) auf Anfrage und nach einer Freigabe seitens des den jeweiligen Dienst nutzenden Nutzers (120) über mindestens eine definierte Schnittstelle auf die mindestens eine jeweilige Auswahl von den auf dem zentralen Datenspeicher (100) gespeicherten Daten des mindestens einen IoT Geräts (141, 142, 151, 152, 153) lesend zugreifen kann.

9. Verfahren nach Anspruch 8, bei dem die Anfrage seitens des jeweiligen Dienstes (180, 190) dem Nutzer auf einer Anzeigeeinheit des Nutzerendgerätes, mit dem der Nutzer den jeweiligen Dienst (180, 190) zuvor über das Telekommunikationsnetz aus dem Internet angefordert hat, angezeigt wird und der Nutzer mittels des Nutzerendgeräts die Freigabe an den zentralen Datenspeicher (100) übermittelt.

10. Verfahren nach Anspruch 9, bei dem der zentrale Datenspeicher (100) nach Eingang der Freigabe für den jeweiligen Dienst (180, 190) einen temporären Zugangsschlüssel für die mindestens eine jeweilige Auswahl von den auf dem zentralen Datenspeicher (100) gespeicherten Daten des mindestens einen IoT Geräts (141, 142, 151, 152, 153) generiert und an das Nutzerendgerät übermittelt, das den temporären Zugangsschlüssel an den jeweiligen Dienst (180, 190) weiterleitet, wobei der temporäre Zugangsschlüssel nach Beendigung der aktiven Sitzung des jeweiligen Dienstes (180, 190) von dem zentralen Datenspeicher (100) ungültig gemacht wird.

11. Anordnung zum Auswerten von IoT Gerätedaten für einen Nutzer, mit mindestens einem IoT Gerät (141, 142, 151, 152, 153) eines ersten Anbieters, das dazu konfiguriert ist, die IoT Gerätedaten zu erfassen und in einem von einem als Übertragungsnetz implementierten Telekommunikationsnetz bereitgestellten zentralen Datenspeicher (100) zu speichern, mit dem zentralen Datenspeicher (100), der dazu ausgelegt ist, die von dem mindestens einen IoT Gerät (141, 142, 151, 152, 153) erfassten IoT Gerätedaten zu speichern und mindestens eine Auswahl der gespeicherten Daten für mindestens einen digitalen Dienst (180, 190) von mindestens einem zweiten Anbieter nach Autorisierung abrufbar zugänglich zu machen.

12. Anordnung nach Anspruch 11, mit dem mindestens einen digitalen Dienst (180, 190) des mindestens einen zweiten Anbieters, wobei der digitale Dienst (180, 190) dazu konfiguriert ist, nach Autorisierung einen Zugang zu der mindestens einen Auswahl von den auf dem zentralen Datenspeicher (100) gespeicherten Daten des mindestens einen IoT Geräts (141, 142, 151, 152, 153) zu erhalten und die Auswahl von Daten abzurufen und die abgerufenen Daten geräteübergreifend zu analysieren, auszuwerten und dem Nutzer mittels eines Nutzerendgerätes bereitzustellen.

13. Anordnung nach einem der Ansprüche 11 oder 12, bei dem der zentrale Datenspeicher (100) dazu ausgelegt ist, eine Mehrzahl von Sektoren (101-1, 101-2, 102-1, 102-2, 102-3) vorzusehen, wobei jeder Sektor (101-1, 101-2, 102-1, 102-2, 102-3) einem jeweiligen IoT Gerät (141, 142, 151, 152, 153) des mindestens einen IoT Geräts (141, 142, 151, 152, 153) zuordenbar ist, und von einem jeweiligen IoT Gerät (141, 142, 151, 152, 153) erfasste IoT Gerätedaten in dem jeweiligen dem jeweiligen IoT Gerät (141, 142, 151, 152, 153) zugeordneten Sektor (101-1, 101-2, 102-1, 102-2, 102-3) abzuspeichern.

14. Anordnung nach einem der Ansprüche 11 bis 13, bei dem der zentrale Datenspeicher (100) dazu konfiguriert ist, nach Eingang einer Freigabe für einen jeweiligen Dienst (180, 190) einen temporären Zugangsschlüssel für von dem jeweiligen Dienst (180, 190) angefragte Daten zu generieren und an das Nutzerendgerät des Nutzers zu übermitteln, das den temporären Zugangsschlüssel an den jeweiligen Dienst (180, 190) weiterleitet, und den temporären Zugangsschlüssel nach Beendigung der Sitzung für einen jeweiligen Dienst (180, 190) ungültig zu machen.

15. Computerprogrammprodukt mit einem computerlesbaren Medium und einem auf dem computerlesbaren Medium gespeicherten Computerprogramm mit Programmcodemitteln, die dazu konfiguriert sind, bei Ablauf des Computerprogramms auf einer Recheneinheit, ein Verfahren nach einem der Ansprüche 1 bis 10, insbesondere unter Verwendung einer Anordnung nach einem der Ansprüche 11 bis 14 auszuführen.
